(19) Europäisches Patentamt
European Patent Office
Office européen des brevets

(11) Publication number: 0 351 350
A1

(12) # EUROPEAN PATENT APPLICATION

(21) Application number: 89710004.6

(22) Date of filing: 13.01.89

(51) Int. Cl.[5]: C 07 D 405/12
A 23 L 1/236, C 07 D 307/87

(30) Priority: 12.07.88 US 216738 11.01.89 US 295390

(43) Date of publication of application:
17.01.90 Bulletin 90/03

(84) Designated Contracting States:
AT BE CH DE ES FR GB GR IT LI LU NL SE

(71) Applicant: THE NUTRASWEET COMPANY (a Delaware corporation)
1751 Lake Cook Road
Deerfield Illinois 60015 (US)

(72) Inventor: Mazur, Robert
523 Maple Avenue
Wilmette, IL. 60091 (US)

Owens, William H.
1214 East Valley Lane
Arlington Heights, IL. 60004 (US)

Klade, Carrie Ann
4544 North Sayre Apt. 2C
Norridge, IL. 60656 (US)

Madigan, Darold
908 Wisconsin Avenue
Elk Grove Village, IL. 60007 (US)

Muller, George W.
1915 Smith Road
Northbrook, IL. 60062 (US)

(74) Representative: Wolff, Hans Joachim, Dr.jur. Dipl.-Chem. et al
Beil, Wolff & Beil Rechtsanwälte Postfach 80 01 40
Adelonstrasse 58
D-6230 Frankfurt am Main 80 (DE)

(54) **High potency sweetening agents.**

(57) The novel sweetening compound 1′-N-[N′-2-Amino-6-oxa-cis-hydrindanyl(4-cyanophenylimino)methyl]-5-Aminomethyltetrazole is disclosed. One isomer having a potency equivalent to about 20,000 the sweetness of sucrose can be substantially isolated. The compound can be used in a wide variety of edible products.

EP 0 351 350 A1

**Description**

## HIGH POTENCY SWEETENING AGENTS

BACKGROUND OF THE INVENTION

The present invention relates to a novel guanidine compound useful as a sweetening agent. This guanidine compound is 1′-N-[N′-2-Amino-6-oxa-cis-hydrindanyl(4-Cyanophenylimino)methyl]-5-Aminomethyltetrazole, particularly the more sweet isomer. Additionally, the present invention relates to sweetening compositions, food products containing the present guanidine, methods of sweetening foods, and methods of preparing the novel guanidine.

Certain guanidine derivatives are known in the art as sweeteners. However certain prior known sweeteners, especially glycine derivatives are unstable due to cyclization.

The present guanidine has been discovered to be more stable than prior art compounds due to the presence of a tetrazole moiety as opposed to carboxyl moieties found in most previously known compounds. The presence of the tetrazole moiety prevents cyclization which was a problem in molecules having a carboxyl. The present guanidine also has a high level of sweetness, which makes it an especially desirable compound. While the guanidine has two isomeric forms, a further advantage is that the more sweet isomer is sufficiently sweet to produce a desirable compound even when not totally isolated from the less sweet isomeric form.

SUMMARY OF THE INVENTION

In accordance with the present invention, a novel sweetening compound is disclosed. This compound is 1′-N-[N′-2-Amino-6-oxa-cis-hydrindanyl(4-Cyanophenylimino)methyl]-5-Aminomethyl tetrazole, particularly the more sweet isomer (for purposes of this application, the isomeric form of the compound having a sweetness equivalent to about 20,000 times the sweetness of sucrose will be referred to as the "more sweet isomer" and the other isomer having a sweetness of less than 400 times the sweetness of sucrose will be referred to as the "less sweet isomer.")

This compound may be used solely or in mixtures with other sweeteners as a sweetening agent in foods, especially carbonated soft drinks.

DETAILED DESCRIPTION OF THE PREFERRED EMBODIMENT

The present substituted guanidine is represented by the following formula:

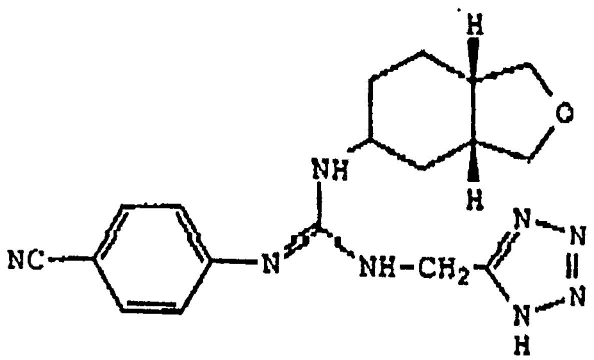

The present invention contemplates both the endo- and exo-forms of the cis-hydrindanyl. It is believed that the endo-isomer is the more sweet isomer. A preferred compound has a substantially purified amount of the more sweet isomer. However, in an isomeric mixture, an amount of the more sweet isomer which is sufficient to produce the sweetness desired for a particular use may be used. For example, if a potency of 10,000 times the sweetness of sucrose is desired, the isomeric mixture need only contain about 50% of the more sweet isomer.

The present invention also includes physiologically acceptable salts of the presented guanidines, i.e., sulfate, phosphate, citrate, hydrochloride, sodium, potassium ammonium, calcium, and magnesium salts.

Additionally, the present invention relates to edible products containing the present compound as sweetening agents either alone or in combination with other sweeteners such as carbohydrate sweeteners or high potency sweeteners. Also provided by the present invention is a process for sweetening edible products such as foods, beverages, candies, chewing gums, sweets, pharmaceuticals, veterinary preparations and the like.

The present invention further contemplates use of the present compound in combination with other sweetening agents and/or a physiologically acceptable carrier which may be a bulking agent. Suitable carriers include polydextrose, starch, maltodextrins, cellulose, methylcellulose, maltitol, carboxymethylcellulose, hydroxymethylcellulose, microcrystalline cellulose, sodium alginate, pectins, gums, lactose, maltose, glucose, leucine, glycerol, mannitol, sorbitol, sodium bicarbonate and phosphoric, citric, tartaric, fumaric, benzoic,

sorbic, propionic acids and their sodium, potassium and calcium salts and mixtures or all of the above.

Suitable sweetening agents which may used in combination with the present compound can be sugars or high potency sweeteners such as sucrose, corn syrups, fructose, high fructose corn syrup, aspartame, alitame, neohesperidine dihydrochalcone, hydrogenated isomaltulose, stevioside, L-sugars, glycyrrhizin, xylitol, acesulfame-K, saccharin (sodium, potassium or calcium salt), trichlorogalactosucrose (TGS or sucralose), monellin, thaumatin, other guanidine sweetening agents, and mixtures thereof.

The present invention also relates to a method of preparing the compound of the present invention, preferably with the more sweet isomer comprising a large amount of the isomeric mixture. The compound may be prepared employing synthetic methods which are analogous to known methods disclosed in the literature. These known methods have been summarized in a paper by Petersen (S. Petersen, Methoden Der Organishe Chemie (Houben-Weyl) Vol. VIII, p.180-8). The techniques are further described in (C. Maryanoff, R.C. Stanzione, J.N. Plampin, and J.E. Mills, J. Org. Chem. 1986, 51, 1882-4); J. Med. Chem., 1978, No. 21, pp. 773-781; Chem. Ber. 1966, 99, 1252-7; U.K. Patent 1587258; J. Org. Chem., 1970, 35, pp. 2067-2069; Chem. Ber. 1967, 100, pp. 591-604; and J. fur Prakt. Chem. 1977, 319, pp. 149-157.

The following example details a method by which the more sweet isomer may be substantially isolated.

SYNTHESIS OF 1′-N-[N′-2-AMINO-6-OXA-CIS-HYDRINDANYL(4-CYANOPHENYLIMINO)METHYL]-5-AMINOMETHYLTETRAZOLE

Step 1: Preparation of 3,4 Dihydroxymethyl cyclohexene:

A solution containing 76.0 g (0.50 moles) of cis-1,2,3,6-tetrahydrophthalic anhydride in 400 ml THF was added dropwise to a suspension containing 25.0 g (0.658 moles) of lithium aluminium hydride at 0° C. After the addition was complete the reaction was allowed to warm to room temperature. After stirring for 1 hr the reaction was diluted with ether, cooled to 0° C and quenched with 50% KOH until a white solid formed. Magnesium sulfate was added and the reaction was filtered. The solid was washed well with ether and concentrated under reduced pressure to a pale yellow oil which was distributed (bp = 126° C/0.6 mm) to give 34.2 g (48%) of a colorless oil which solidified (mp = 37-38° C) when stored in the refrigerator. PMR ($CDCl_3$) δ 5.61 (s, 2H), 4.58 (br s, 2H), 3.73-3.60 (m, 2H), 3.60-3.48 (m, 2H) and 2.20-1.95 (m, 6H). CMR ($CDCl_3$) δ 125.5, 63.6, 37.8 and 26.9. IR (neat) 3329, 3025, 2892, 1438 and 957 $cm^{-1}$.

Step 2: Preparation of 6-Oxa-cis-2-hydrindene:

The diol obtained above (25.8 g, 182 mmoles) was dissolved in 150 ml of DMSO and heated to reflux while exposed to air. After heating overnight, the reaction was cooled, diluted with water and extracted twice with petroleum ether. The combined petroleum ether extracts were dried with magnesium sulfate and concentrated under reduced pressure to give a dark orange liquid. Distillation gave 15.5 g (69%) of a colorless liquid (bp = 75-77° C/18 mm). PMR ($CDCl_3$) δ 5.69 (t, J = 1.6 Hz, 2H), 3.89 (dd, J = 7.8, 6.2 H, 2H), 3.54 (dd, J = 7.8, 5.6 Hz, 2H), 2.43-2.16 (m, 4H) and 2.03-1.89 (m, 2H). CMR ($CDCl_3$) δ 124.9, 73.2, 35.3 and 24.1. IR (neat) 3025, 2928, 2855, 1437, 1135, 1122 and 1056 $cm^{-1}$.

Step 3: Preparation of 6-Oxa-cis-hydrindan-2-ol:

A solution containing 4.0 g (32.3 mmoles) of the olefin prepared above was cooled to 5° C and treated with 11.8 mL (11.8 mmoles) of 1M borane in THF. When the addition was compete the ice bath was removed and the reaction was stirred at room temperature for 1 hr. Excess borane was destroyed by the careful addition of water until gas evolution ceases followed by the addition of 4.1 mL (12.3 mmoles) of sodium hydroxide and the dropwise addition of 4.1 mL (36.2 mmoles) of 30% $H_2O_2$ with cooling in an ice bath as needed. The reaction is then heated to 50° C for 1 hr to ensure complete oxidation. After cooling and extraction with ether, the ether layer is dried and concentrated under reduced pressure to give 2.6 g (56%) of a colorless liquid. The aqueous layer was reextracted with $CHCl_3$ (2X) to give an additional 2.1 g (44%) of alcohol that was combined with the initial product (total yield = 100%). It was evident by NMR that this alcohol was a mixture of exo and endo isomers. PMR ($CDCl_3$) δ 3.9-3.77 (m, 3H), 3.70-3.55 (m, 2H), 2.78 (br s, 1H), 2.58-2.10 (m, 2H) and 1.93-1.28 (m, 6H). CMR ($CDCl_3$) δ (major isomer) 72.6, 70.8, 66.1, 37.5, 37.3, 32.7, 32.3 and 23.3. (minor isomer) 74.2, 69.2, 69.0, 38.5, 36.5, 35.6, 30.1 and 21.9. IR (neat) 2932, 2865, 1488, 1451, 1125, 1074, 1023 and 887 $cm^{-1}$.

Step 4: Preparation of 6-Oxa-cis-hydrindan-2-one:

To a suspension containing 10.5 g (48.7 mmoles) of pyridinium chlorochromate and 15 g of celite in 90 mL of methylene chloride was added rapidly, in one portion, a solution containing 4.6 g (32.4 mmoles) of the alcohol from above in 10 ml methylene chloride. The reaction immediately turned dark brown. After stirring for 3 hrs the reaction was diluted with ether and filtered through a pad of florosil. The filtrate was concentrated under a reduced pressure to give 3.85 g (85%) of a pale yellow oil. PMR ($CDCl_3$) δ 4.0-3.9 (m, 2H), 3.7-3.6 (m, 1H), 3.54-3.46 (m, 1H), 2.74-2.16 (m, 6H), 2.12-1.97 (m, 1H) and 1.90-1.76 (m, 1H). CMR ($CDCl_3$) δ 212.0, 73.7, 73.4, 40.9, 37.6, 37.5, 36.7 and 24.9. IR (neat) 2932, 2854, 1720, 1480, 1460, 1418, 1324, 1245, 1079, 1045 and 926 $cm^{-1}$.

Step 5: Preparation of 6-Oxa-cis-hydrindan-2-oxime:

A solution containing 4.1 g (29.3 mmole) of the ketone prepared above in 25 ml absolute ethanol was treated with 2.04 g (29.3 mmole) of hydroxylamine HCl in 10 mL water followed by 2.03 g (14.7 mmole) of potassium carbonate in 15 mL water. The reaction was heated to reflux and stirred for 2 hr. After cooling the reaction was concentrated under reduced pressure and extracted with ether. The ether layer was dried and concentrated under reduced pressure to give 3.9 g (87%) of a pale yellow oil shown by NMR to be a mixture of syn and anti isomers. PMR ($CDCl_3$) δ 9.80 (br s, 1H), 3.95-3.87 (m, 2H), 3.72-3.46 (m, 2H), 2.7-2.50 (m, 1H), 2.57-2.28 (m, 4H), 2.26-2.12 (m, 1H), 1.95-1.77 (m, 1H) and 1.70-1.50 (m, 1H). CMR ($CDCl_3$) δ (major isomer) 159.6, 73.6, 73.5, 37.3, 37.1, 30.2, 23.3 and 22.8. (minor isomer) 158.6, 72.6, 72.2, 37.65, 37.60, 28.4, 25.5 and 23.1.

Step 6: Preparation of 2-Amino-6-oxa-cis-hydrindane:

A solution containing 3.8 g (24.5 mmole) of the oxime prepared above in 30 mL anhydrous THF was added to a suspension of 2.81 g (73.9 mmole) of lithium aluminum hydride in 70 mL anhydrous THF at 0° C. The reaction was heated to reflux and stirred overnight. After cooling to 0° C and diluting with ether, the reaction was quenched using 6N KOH until a white solid formed. The solid was filtered and washed well with ether. The filtrate was concentrated under reduced pressure to give 3.2 g (94%) of a yellow oil shown by NMR to be a 1:1 mixture of endo/exo isomers. PMR ($CDCl_3$) δ 3.90-3.75 (m, 2H), 3.69-3.58 (m, 2H), 2.90-2.80 (m, 0.5H), 2.72-2.60 (m, 0.5H), 2.56-2.00 (m, 2H), 1.90-1.30 (m, 7H) and 1.20-1.05 (m, 1H). CMR ($CDCl_3$) δ (major isomer) 74.5, 68.6, 49.4, 38.8, 37.1, 36.4, 31.1 and 22.6. (minor isomer) 73.7, 69.9, 45.6, 38.0, 37.8, 34.7, 33.7 and 25.3. IR (neat) 3351, 2928, 2862, 1598, 1449, 1054 and 891 $cm^{-1}$.

Step 7: Preparation of N-2-(6-oxa-cis-hydrindanyl)-N′-4-Cyanophenyl Thiourea:

4-Cyanophenylisothiocyanate (4.0 g; 25.0 mmol) was dissolved in 75 mL ethyl acetate. 2-Amino-6-oxa-cis-hydrindane (3.1 g, 22.0 mmol) prepared above was added to the reaction mixture with stirring at room temperature. After 10 minutes, a precipitate formed. The reaction mixture was stirred overnight and filtered to give 3.5 g (53%) of white solid. This solid was recrystallized from acetonitrile to give 1.9 g of product enriched in the endo isomer. This sample was triturated with hot acetonitrile and filtered after cooling to give 1.0 g (15%) of a white solid shown by HPLC (30% $CH_3CN$) to be >98% pure. mp = 195-196° C. PMR (DMSO-D6) δ 9.79 (br s, 1H), 8.08 (m, 1H), 7.74 (s, 4H), 4.18-4.02 (m, 1H), 3.78-3.66 (m, 2H), 3.6-3.48 (m, 2H), 2.4-2.15 (m, 2H), 1.96-1.69 (m, 4H) and 1.43-1.18 (m, 2H). CMR (DMSO-D6) δ 178.7, 144.2, 132.6, 120.9, 119.1, 104.3, 73.5, 67.7, 51.6, 37.8, 35.8, 26.0 and 21.9.

Step 8: Synthesis of N-2-(6-Oxa-cis-hydrindanyl)-N′-4-Cyanophenyl-S-Methylisothiourea:

A mixture of 1.0 g (3.32 mmol) of the thiourea obtained above and 0.52 mL (d = 2.28; 8.35 mmol) of methyl iodide was stirred overnight in 20 mL of acetone. In the morning, a solid was present and it was filtered to give 1.35 g (90%) of a white solid. The solid produced was dissolved in 5 mL of a 1N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 0.95 g (95%) of a white solid (mp = 150-151.5° C). PMR ($CDCl_3$) δ 7.54-6.93 (AB q, J = 8.4 Hz, 4H), 4.59 (br s, 1H), 3.88-3.60 (m, 5H), 2.46-2.74 (m, 1H), 2.24 (s, 3H) 2.08-1.750 (m, 5H) and 1.34-1.17 (m, 2H). CMR ($CDCl_3$) δ 154.2, 153.0, 133.1, 123.0, 119.8, 105.0, 74.4, 68.6, 50.8, 38.5, 36.3, 33.3, 27.5, 22.4 and 14.2.

Step 9: Preparation of 1′-N-[N-2-Amino-6-oxa-cis-hydrindanyl(4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 mL of water was added to a soluton of N-2-(6-oxa-cis-hydrindanyl)-N′-4-cyanophenyl-S-methylisothiourea (0.95 g, 3.02 mmol) in 15 mL of absolute ethanol. The mixture was heated to reflux for 20 hours. After cooling, the solution was concentrated to dryness and the residue was dissolved in 15 mL of water. The resulting aqueous solution was washed with ether (2 x 20 mL) and then neutralized with a 1 N HCl solution to bring the pH to 7.5. The desired guanidine compound precipitated and was separated out by filtration to give 0.87 g (79%) of the title compound. PMR (DMSO-D6) δ 7.87-7.41 (AB q, J = 8.5 Hz, 4H), 4.60 (s, 2H), 3.74-3.44 (m, 5H), 2.36-2.22 (m, 1H), 2.22-2.10 (m, 1H), 1.89-1.62 (m, 4H) and 1.50-1.20 (m, 2H). CMR (DMSO-D6) δ 158.1, 153.2, 142.8, 133.6, 122.1, 118.8, 106.0, 73.4, 67.5, 50.4, 37.6, 35.4, 34.2, 26.3 and 21.6. IR (KBr) 2238, 1658, 1626, 1600, 1577 and 1510 $cm^{-1}$. Analysis calculated for $C_{18}H_{22}N_8O$ (1.1 $H_2O$): C, 55.98; H, 6.31; N, 29.01. Found: C, 55.84; H, 6.28; N, 28.89. The compound was analyzed by HPLC and was found to include more than 99% of the more sweet isomer of the compound (believed to be the endo- isomer). The compound was tasted and was found to have a sweetness equivalent to about 20,000 times the sweetness of sucrose.

## Claims

1. A compound corresponding to the formula:

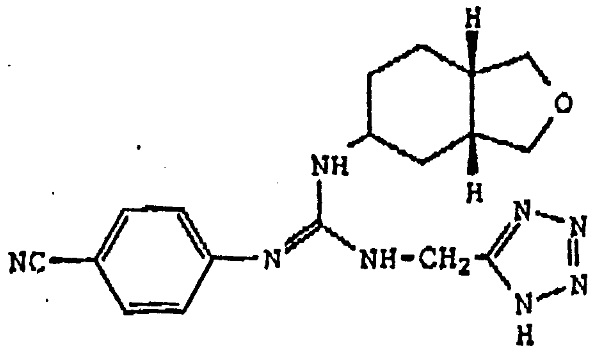

and tautomers and physiologically acceptable salts thereof.

2. The compound of Claim 1 comprising substantially the more sweet isomer of said compound.

3. The compound of Claim 1 wherein said more sweet isomer is present in an amount of at least 50%.

4. The compound of Claim 1 wherein said more sweet isomer is present in an amount sufficient to impart a desired level of sweetness.

5. A process for sweetening edible products including foods, beverages, confections, chewing gums, pharmaceuticals, veterinary preparations and toilet, cosmetic, and hygiene products, characterized in that it comprises adding to the substance an effective sweetening amount of the compound of Claim 1.

6. Edible products sweetened according to the process of Claim 5.

7. Sweetening compositions characterized in that they comprise an effective sweetening amount of the compound of Claim 1 and a physiologically acceptable carrier therefor.

8. The sweetening composition of Claim 7 wherein the carrier is a bulking agent.

9. The sweetening composition of Claim 7 wherein the carrier is selected from the group consisting of polydextrose, starch, maltodextrins, cellulose, methylcellulose, carboxymethylcellulose, maltitol, hydroxymethylcellulose, microcrystalline cellulose, sodium alginate, pectins, gums, lactose, maltose, glucose, leucine, glycerol, mannitol, sorbitol, sodium bicarbonate, and phosphoric, citric, tartraric, fumaric, benzoic, sorbic, propionic acids and their sodium, potassium and calcium salts and mixtures of any of the above.

10. A sweetening composition comprising:

(a) the compound of Claim 1; and

(b) a different sweetening agent.

11. The sweetening composition of Claim 10 further comprising a carrier.

12. The sweetening composition of Claim 10 wherein said different sweetening agent is selected from the group consisting of sucrose, corn syrups, fructose, aspartame, alitame, neohesperidine dihydrochalcone, high fructose corn syrup, hydrogenated isomaltulose, stevioside, L-sugars, glycyrrhizin, xylitol, acesulfam-K, saccharin (sodium potassium or calcium salt), cyclamic acid (sodium, potassium or calcium salt), trichlorogalactosucrose, monellin, thaumatin, guanidines, and mixtures thereof.

13. A process comprising: reacting N-2-(6-Oxa-cis-hydrindanyl)-N′-4-Cyanophenyl-S-Methylisothiourea with 5-aminomethyltetrazole to produce 1′-N-[N′-2-Amino-6-oxa-cis-hydrindanyl(4-cyanophenylimino)methyl]-5-Aminomethyltetrazole.

14. The process of Claim 13 wherein said 5-Aminomethyltetrazole is reacted with substantially the isomer of said N-2-(6-oxa-cis-hydrindanyl)-N′-4-Cyano- phenyl-S-Methylisothiourea capable of producing the isomer of 1′-N-[N′-2-Amino-6-oxa-cis-hydrindanyl(4-cyano-phenylimino)methyl]-5-Aminomethyltetrazole having a sweetness about 20,000 the sweetness of sucrose.

15. 2-Amino-6-ox-cis-hydrindane.

16. The compound of Claim 15 comprising substantially the isomer of 2-Amino-6-oxa-cis-hydrindane capable of producing the more sweet isomer of 1′-N-[N′-2-Amino-6-oxa-cis-hydrindanyl(4-cyanophenylimino)methyl]-5-Aminomethylterrazole.

European Patent Office

# EUROPEAN SEARCH REPORT

Application Number

EP 89 71 0004

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 195 730 (UNIVERSITE CLAUDE BERNARD)<br>* claims; abstract * | 1,5-12 | C 07 D 405/12<br>A 23 L 1/236<br>C 07 D 307/87 |
| A | EP-A-0 034 925 (ELI LILLY AND CO)<br>* cliams 1,8,19 * | 1,7,10,12 | |
| A | US-A-3 294 551 (R.M. HERBST)<br>* columns 1,2, line 63 * | 1,7 | |
| D,A | GB-A-1 587 258 (AKTIESELSKABET GEA)<br>* page 1, lines 21-33 * | 13 | |
| P,A | CHEMICAL ABSTRACTS vol. 109, no. 21, 21st November 1988, page 688, abstract no. 190159y, Columbus, Ohio, US; N.S. KOZLOV et al.:"Synthesis of 3ar,5t,6t,7ac-perhydro-5,6-epoxyisobenzo furan and the study of its reactions with amines and alcohols."; & Vestsi Akad. Navuk BSSR, Ser. Khim. Navuk 1987,(6), 58-62 | 15 | |

TECHNICAL FIELDS SEARCHED (Int. Cl.4)

A 23 L 1/00
C 07 D 257/00
C 07 D 307/00
C 07 D 405/00

The present search report has been drawn up for all claims

| Place of search | Date of completion of the search | Examiner |
|---|---|---|
| BERLIN | 29-03-1989 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)